# EUROPEAN PATENT APPLICATION

(11) **EP 2 083 082 A1**
(43) Date of publication of application: **29.07.2009**
(21) Application number: 08001399.8
(22) Date of filing: 25.01.2008
(51) Int. Cl.: C12N 15/82, A01H 5/00

(54) **Overproduction of vitamin B6 in plants**

(71) Applicant: ETH Zurich, 8092 Zürich (CH)
(72) Inventor: Fitzpatrick, Teresa, 8048 Zürich (CH); Raschke, Maja, 8046 Zürich (CH); Amrhein, Nikolaus, 8038 Zürich (CH)

(57) **Abstract**

The invention relates to a method of overproduction of vitamin B6 in plants by introducing endogenous genes *PDX1* and optionally *PDX2* into said plants, and to plants and plant parts so produced. Overexpression of either *PDX1* alone or in combination with *PDX2* leads to a substantial increase in the vitamin B6 content in green parts and seeds of the plants. Seeds of such plants are enlarged compared to wild-type seeds as a result of increased carbohydrate, lipid and protein content.

## Description

### Field of the invention

The invention relates to a method of overproduction of vitamin B6 in plants, and to plants and plant parts overproducing vitamin B6.

### Background of the invention

Vitamin B6 is an essential metabolite in all organisms in that it can act as a cofactor for numerous metabolic enzymes. Its *de novo* biosynthesis occurs only in bacteria, fungi and plants, making it an essential nutrient in the human diet. The major source of vitamin B6 for humans is from plants. However, it has recently been recognized that many people are deficient in vitamin B6 even in Western society. Deficiency of the vitamin is associated with many illnesses, for example, in the field of nervous and immune system function, red blood cell formation (heme biosynthesis), hormone function, as well as atherosclerosis and other cardiovascular diseases. Recently, a completely unprecedented function of vitamin B6 as an antioxidant was uncovered, with a potency equivalent to that of vitamins C and E. Moreover, there is growing evidence that the vitamin may also have a novel role as an anti-tumor agent. Thus, there is an interest to overproduce the vitamin for nutrient and other beneficial effects, e.g. medicinal purposes. Much of vitamin B6 used for supplementation purposes is currently produced chemically. However, bottlenecks imposed by low yields, poor and inconsistent product quality and difficulties with downstream processing pose a challenge in this field.

Plants are now becoming accepted as a platform to produce nutrients either for extraction to be used in industry or directly in crop plants to increase nutritional content. Transgenic expression of vitamin biosynthesis genes has been reported for vitamins A, C, E and B9. In order to increase the nutrient content of food the pathway of biosynthesis of the compound in question needs to be known. It was tacitly assumed that the vitamin B6 biosynthesis pathway in plants was the same as that in the Gram-negative bacterium *Escherichia coli,* the workhorse of molecular biology. However, it has very recently been demonstrated that this is not the case and a completely novel pathway has since been unravelled. The pathway is characterized by the presence of two genes, namely *PDX1* and *PDX2.* The corresponding proteins PDX1 and PDX2 are responsible for vitamin B6 biosynthesis in plants and moreover both are essential for plant viability. The latter point is also of interest in the context of the pathway being exploited as a herbicide target. Studies describing the pathway and the essentiality of PDX1 and PDX2 have been published (Tambasco-Studart, M. et al., PNAS 102 (2005) 13687-13692, and Tambasco-Studart, M. et al., Plant Physiol. 144 (2007) 915-925; Titiz, O. et al., Plant J. 48 (2006) 933-946).

In a recent publication attempts to overproduce vitamin B6 in tobacco have been reported, but the attempt met with failure except in one single cell line where a slight overproduction could be observed. It was speculated that native genes were down-regulated by the introduction of exogenous genes *PDX1* and *PDX2,* and it was concluded that it is not possible to overproduce vitamin B6 in plants (Herrero S. and Daub, M.E., Plant Science 172 (2007) 609-620).

### Summary of the invention

The invention relates to a method of overproduction of vitamin B6 in plants by introducing the endogenous gene *PDX1,* optionally in combination with the endogenous gene *PDX2,* into said plants. Furthermore the invention relates to plants and plant parts so produced overexpressing vitamin B6, in particular seeds of such plants.

In further aspects, the invention relates to the use of plants or plant parts, in particular seeds, for the treatment of pathological conditions related to an overproduction of free radicals, in particular diseases connected with aging, artherosclerosis and cancer.

In yet a further aspect the invention relates to a method of increasing stress resistance of plants by the method of the invention, i.e. by introducing endogenous genes *PDX1* and optionally *PDX2* into said plants.

The method of the invention leads to a substantial increase in the vitamin B6 content in green parts and seeds of the plants. Seeds of such plants are enlarged compared to wild-type seeds as a result of increased carbohydrate, lipid and protein content.

Thus, a further aspect of the invention is the overexpression of the endogenous gene *PDX1,* optionally in combination with *PDX2* to increase seed yield and quality.

### Brief description of the figures

**Figure 1****.** Resistance of *Arabidopsis* plants enhanced in vitamin B6 content compared to wild-type plants. Lines overexpressing either PDX1 alone or in combination with PDX2 are shown in the presence (right, PQ) and absence of paraquat (left, control C). WT: wild-type.
**Figure 2****.** Morphology of seeds enhanced in vitamin B6 content compared to wild-type (WT). The top panel A shows seeds of the respective lines, and the bottom panel B is an enlarged picture to emphasize the increase in seed size.

### Detailed description of the invention

The invention relates to a method of overproduction of vitamin B6 in plants, and to plants so produced, in particular seeds of such plants. Specifically, the invention relates to plant parts including seeds having at least double the content of vitamin B6 when compared to wild-type plant parts, preferably at least three times the content.

Overproduction of vitamin B6 in plants is accomplished by introducing the endogenous gene *PDX1,* optionally in combination with the endogenous gene *PDX2,* into said plants. In the following text a practical procedure is described how to proceed in Arabidopsis, but the invention is not restricted to Arabidopsis: PDX1 and PDX2 from Arabidopsis were separately cloned into the plant binary vectors pCAMBIA 1300.1 and pBIN19, to give pCAM-AtPDX1 and pBINAtPDX2, respectively. The pCAM-AtPDX1 and pBIN-AtPDX2 constructs were then transformed into the bacterium *Agrobacterium tumefaciens*, which facilitates delivery of the T-DNA part of the construct into the plant cell. The T-DNA that contains *PDX1* or *PDX2* is inserted randomly into the Arabidopsis genome. The expression of the individual genes is driven in a constitutive and universal fashion by the cauliflower mosaic virus 35S promoter (CaMV35S). In the described technology, homozygous lines overexpressing *PDX1* were crossed to lines overexpressing *PDX2* to give lines overexpressing both genes. It is also possible to incorporate both *PDX1* and *PDX2* into the same construct and proceed in the same way, with no necessity for crossing individual plant lines.

The modified plants so prepared differ from non-modified plants in that they contain an extra piece of DNA that includes the *PDX* gene(s), the CaMV35S promoter and an antibiotic resistance gene. The invention requires that plants contain the extra *PDX* gene(s). However, the presence of the particular CaMV35S promoter and the particular antibiotic resistance marker gene is not decisive for the success of the procedure. Any promoter, and, if desired, any type of marker gene, may be used in combination with the *PDX* genes.

While the experiments to demonstrate the success of the method of invention have been carried out with *Arabidopsis* plants, the invention is not restricted to these. The same technology can be applied to crop plants by cloning the endogenous genes of the crop plant of interest into the appropriate vector for transformation into said crop plant. It is noteworthy in this context that it is relatively easy to identify homologs of *PDX* genes as they are very highly conserved in evolution.

The method of the invention relates particularly to a method wherein endogenous genes *PDX1* and optionally *PDX2* are isolated, introduced into a plant vector under control of an efficient promoter, inserted into the plant genome, and plants regenerated.

Examples of useful plant vectors are pCAMBIA, pBIN, pBI101, pMDC, pORE, and pPZPY. Efficient promoters are, for example, CaMV35S, ubiquitin promoter, FMV promoter, V-ATPase promoter, and Glutelin (Gt1, e.g. rice endosperm specific).

Plants considered according to the invention are any plants, in particular crop plants. Preferred crop plants are, for example, wheat, barley, corn, rice, potato, cassava, sweet potato, tomato, and soybean. Most preferred are rice, sweet potato and tomato.

Overexpression of either PDX1 alone, or PDX1 and PDX2 in combination, leads to an increase in the total vitamin B6 content (Table 1).

**Table 1: Characteristics of Arabidopsis plants overproducing proteins involved in vitamin B6 biosynthesis, either PDX1 or PDX2 alone or both together, compared to wild-type grown under dark light cycle.**

| | **Wild-type** | **AtPDX1-OE** | **AtPDX2-OE** | **AtPDX1-AtPDX2-OE** |
|---|---|---|---|---|
| Weight of seeds [µg/seed] | 20.6 ± 2.8 | 32.4 ± 1.7 | 19.0 ± 1.2 | 35.7±2.19 |
| Total B6 content, **shoots** [ng/mg FW] | 3.4 ± 1.1 | 9.7 ± 2.7 | 3.1 ± 0.9 | 11.4±1.2 |
| Total B6 content, **roots** [ng/mg FW] | 3.1 ± 1.1 | 3.8 ± 1.7 | 1.8 ± 0.6 | 5.0±0.3 |
| Total B6 content, **seeds** [ng/mg FW] | 6.8 ± 1.4 | 24.9 ± 2.4 | 4.9 ± 0.1 | 36.9±1.4 |

| | | | | |
|---|---|---|---|---|
| FW: fresh weight | | | | |

Importantly, the accumulation of the vitamin appears to be in both green tissue and seeds.

The term vitamin B6 refers to pyridoxine, pyridoxamine, pyridoxal and their phosphorylated derivatives. The active cofactor form is pyridoxal 5'-phosphate. The main form accumulated in the modified plants according to the invention is pyridoxal 5'-phosphate and pyridoxine.

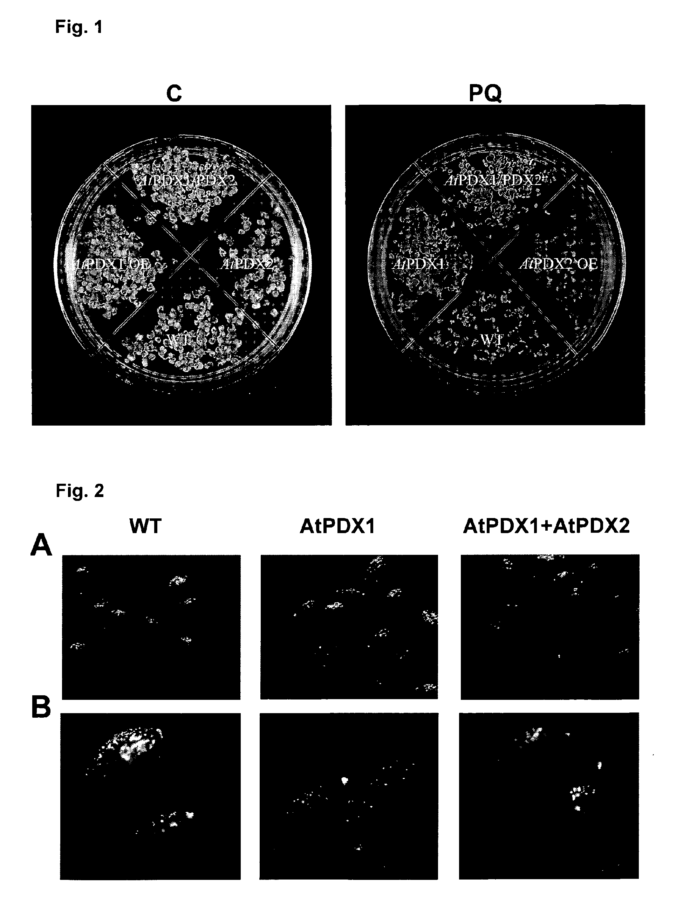

In addition, an unprecedented consequence of overproducing the vitamin is that the seeds of the modified plants are substantially enlarged (Figure 2). In particular, the seeds of plants that overexpress both PDX1 and PDX2 show an approximate 180% increase in mass (Table 2). This appears to be reflected in enriched protein, lipid and carbohydrate content (Table 2). Specifically, the carbohydrate content (both soluble and insoluble) appears to be the largest contributor to the increase in mass. As seeds and fruits are a main resource for both human consumption and animal feed, there are many ongoing projects worldwide that aspire to increase seed biomass and yield. The features of increased biomass observed with *Arabidopsis* plants can be exploited to achieve the same or closely related increases in seed mass and vitamin B6, carbohydrate, protein content in crop plants by introducing the *PDX* genes into such crop plants in the appropriate way. It must be mentioned that plant seeds are especially attractive as production units. In addition to a high production capacity, they offer other important advantages over other parts of the plant. The seeds can be stored for a long time without losing the produced nutrients effectiveness, so that a reserve can always be kept on hand.

This means that the nutrients can be isolated from the seeds at the moment that they are actually needed.

Based on the observations in Table 2 and Figure 2, the invention also relates in particular to seeds having at least 50% more average mass than wild type, and to seeds having at least 50% more protein content, at least 40% more lipid content and at least 50% more carbohydrate content, more particularly to seeds having at least 70% or 100% more carbohydrate content.

**Table 2: Characteristics of seeds of Arabidopsis plants overproducing proteins involved in vitamin B6 biosynthesis, either PDX1 alone or PDX1 and PDX2 together, compared to wild-type.**

| | **Seed FW** | **Seed DW** | ***% Total*** | ***% Total*** | ***%Total*** | **% *Total*** |
|---|---|---|---|---|---|---|
| | **(µg)** | **(µg)** | ***protein*** | ***lipids*** | ***sol. ch*** | ***insol. ch*** |
| **WT** | 20.6 ± 2.8 | 12.9 ± 0.3 | 100.0 | 100.0 | 100.0 | 100.0 |
| **AtPDX1** | 32.4 ± 1.7 | 19.3 ± 1.7 | 161.7 | 142.4 | 175.4 | 157.4 |
| ***At*PDX1/AtPDX2** | 35.7 ± 2.2 | 19.8 ± 1.0 | 184.7 | 168.5 | 208.6 | 164.4 |

| | | | | | | |
|---|---|---|---|---|---|---|
| ch: carbohydrate, sol.: soluble, insol.: insoluble, FW: fresh weight, DW: dry weight. Values were determined and calculated according to Focks, N. and Benning, C., Plant Physiology 118 (1998) 91-101 [protein], Siloto, R.M.P. et al., Plant Cell 18 (2006) 1961-1974 [lipids], and Smith A.M. and Zeeman, S., Nature Protocols 1 (2006) 1342-1345 [carbohydrates]. | | | | | | |

The invention further relates to the use of plants or plant parts of the invention, in particular seeds, for the treatment of pathological conditions related to an overproduction of free radicals, in particular diseases connected with aging, artherosclerosis and cancer.

Such use may be by direct consumption of plant parts, for example seeds, or preparing extracts containing increased amounts of vitamin B6, to be used as food additives or as pharmaceutical preparations, in particular pharmaceutical preparations for oral use, for example in the form of tablets, capsules, or also in the form of aqueous solutions or suspensions to be used in drinkable forms, for example as a syrup, or as a solution used as drops. Alternatively, plants overexpressing endogenous *PDX* genes may be used for industrial production of vitamin B6, extracting it from plants according to known procedures of the state of the art and applying vitamin B6 as medicaments against aging, artherosclerosis and cancer in standard pharmaceutical compositions, for example as tablets, capsules, solutions or suspensions.

The invention further relates to the method of the invention as a method of increasing stress resistance of plants, characterized by introducing genes *PDX1* and optionally *PDX2* into said plants.

Stress conditions considered are oxidative stress, stress caused by UV-B, and salt stress, in particular oxidative stress. Application of the herbicide paraquat to plants induces oxidative stress, since paraquat produces reactive oxygen in the form of superoxide. Figure 1 demonstrates increased tolerance to paraquat and hence to oxidative stress.

The following example serves to illustrate the invention without limiting the invention in its scope.

### Example

Vector construction: *AtPDX1.1* (At2g38230) was amplified by PCR using the following primers: 5'-GGGGTACCATGGCAGGAACCGGAGTTGTGG-3' (SEQ ID NO:1, Kpnl restriction site included) and 5'-GCTCTAGATTACTCAGAACGACTAGCGAACC-3' (SEQ ID NO:2, Xbal restriction site included), *AtPDX2* (At5g60540) was amplified using 5'-GGTACCATGACCGTCGGAGTTTTAGCTTTG-3' (SEQ ID NO:3, EcoRI restriction site included) and 5'-GAATTCTTATTGAAATATAGGAAGATCAGGC-3' (SEQ ID NO:4, BamHI restriction site included) as primers and isolated cDNA as a template. PCR was performed under the following conditions: 94°C, 2 min followed by 35 cycles of 94°C, 45 sec; 55°C, 45 sec; 72°C, 1.5 min; 72°C, 7 min. Pfu polymerase (Life Invitrogen) was used for gene amplification. The purified PCR products were subcloned into the commercially available PCR 2.1 TOPO^{®} vector (Topo TA Cloning Kit, Invitrogen) according to the manufacturer's recommendations and subsequently cloned into the plant binary vector pCAMBIA1300.1 (AtPDX1.1), and pSH9 (AtPDX2), respectively, using the restriction sites indicated above and confirmed by sequencing. A cassette containing a single CaMV 35S promoter and AtPDX2 of pSH9 was cloned into the final destination vector pBIN19 using the HindIII restriction site. The resulting vectors (pCAM-AtPDX1.1, pBIN-AtPDX2) were transformed into *Agrobacterium tumefaciens* C58 to be used for plant transformation.

Transformation of *Arabidopsis thaliana*. Transformation of *Arabidopsis thaliana* (ecotype Columbia Col-0) was performed using the floral dip method (Clough, S.J. and Bent, A.F. Plant J. 16 (1998) 735-743).

Selection of transgenic lines. Seeds of the transformed plants were selected for resistance to either hygromycin (AtPDX1.1) or kanamycin (AtPDX2) on Murashige & Skoog (MS) medium including vitamins (Duchefa). Homozygote plants of the fourth generation (T4) carrying a single insertion of the gene cassette were analyzed. At least five independent lines were analysed for their potential to overexpress either PDX1.1 or PDX2. In both cases, the lines carrying the highest accumulation of either protein were used and termed AtPDX1.1-OE and AtPDX2-OE, respectively.

Establishing an AtPDX1.1-AtPDX2 co-overexpressing line. Homozygous plants of the fourth generation of AtPDX1.1- and AtPDX2-OE, respectively, were cultivated on soil and subsequently crossed with each other. Both directions of crossing (fAtPDX1.1-OE - mAtPDX2-OE, mAtPDX1.1-OE - fAtPDX2-OE) were performed and subsequently analysed without showing any difference in phenotype. Analyses were performed using the cross fAtPDX1.1-OE - mAtPDX2-OE. Seeds of the crossed lines were selected for both hygromycin and kanamycin resistance on MS medium as indicated above. Homozygote lines for both of the genes were analyzed for their ability to accumulate PDX1.1 and PDX2 at both the RNA and protein level. The line used for analyses is annotated AtPDX1-PDX2-OE.

## Claims

1. A method of overproduction of vitamin B6 in plants by introducing endogenous gene *PDX1,* optionally in combination with endogenous *PDX2,* into said plants.

2. The method according to claim 1 wherein endogenous genes *PDX1* and optionally *PDX2* are isolated, introduced into a plant vector under control of an efficient promoter, inserted into the plant genome, and plants regenerated.

3. The method according to claim 1 or 2 wherein both endogenous genes *PDX1* and *PDX2* are introduced into said plants.

4. The method according to any one of claims 1 to 3 wherein the plant is wheat, barley, corn, rice, potato, sweet potato, tomato, and soybean.

5. A method of increasing stress resistance of plants by introducing endogenous genes *PDX1,* optionally in combination with endogenous *PDX2,* into said plants.

6. A plant overexpressing vitamin B6.

7. A plant according to claim 6 which is wheat, barley, corn, rice, potato, sweet potato, tomato, and soybean.

8. Seeds of a plant according to claim 6 or 7 which are enlarged compared to wild-type seeds as a result of increased carbohydrate, lipid and protein content.

9. Use of plants or plant parts overexpressing vitamin B6 for the treatment of pathological conditions related to an overproduction of free radicals.

10. Use according to claim 9 wherein the pathological condition is a disease connected with aging, artherosclerosis and cancer.


SEQUENCE LISTING

<110>  ETH Zürich

<120>  Overproduction of Vitamin B6 in Plants

<130>  P

<160>  4

<170>  PatentIn version 3.4

<210>  1
<211>  30
<212>  DNA
<213>  Artificial

<220>
<223>  AtPDX1.1 probe with KpnI site

<400>  1
ggggtaccat ggcaggaacc ggagttgtgg                                        30

<210>  2
<211>  31
<212>  DNA
<213>  Artificial

<220>
<223>  AtPDX1.1 probe with XbaI site

<400>  2
gctctagatt actcagaacg actagcgaac c                                      31

<210>  3
<211>  29
<212>  DNA
<213>  Artificial

<220>
<223>  AtPDX2 probe with EcoRI site

<400>  3
ggtaccatga ccgtcggagt tttagcttt                                         29

<210>  4
<211>  31
<212>  DNA
<213>  Artificial

<220>
<223>  AtPDX2 probe with BamHI site

<400>  4
gaattcttat tgaaatatag gaagatcagg c                                      31